# EUROPEAN PATENT APPLICATION

(11) **EP 4 843 573 A1**
(43) Date of publication of application: **25.11.2026**
(21) Application number: 26186699.0
(22) Date of filing: 06.02.2017
(51) Int. Cl.: A61K 31/439, A61K 9/20, A61K 9/48, A61P 25/34

(54) **SALT**

(30) Priority: 05.02.2016 GB 201602145
(62) Document number(s) of the earlier application(s) in accordance with Art. 76 EPC: 23197173.0 / 4 265 298; 17704814.7 / 3 411 029
(71) Applicant: Achieve Pharma UK Limited, Henley-on-Thames, Oxfordshire RG9 1AY (GB)
(72) Inventor: CLARKE, Anthony., Henley-on-Thames, Oxfordshire RG9 1AY (GB); STEWART, Richard Allistair., Henley-on-Thames, Oxfordshire RG9 1AY (GB); WILLIS, Andrew Gareth., Henley-on-Thames, Oxfordshire RG9 1AY (GB)
(74) Representative: Adamson Jones

(57) **Abstract**

A succinate salt of cytisine. Pharmaceutical compositions comprising a succinate salt of cytisine, for oral, nasal, or topical administration. Pharmaceutical compositions in solid form. comprising a succinate salt of cytisine. Pharmaceutical compositions in unit dosage form. comprising a succinate salt of cytisine.

## Description

The present application relates to a novel salt of cytisine and to pharmaceutical compositions comprising a novel cytisine salt.

Cytisine is a pyridine-like alkaloid known to be a potent nicotinic acetylcholine receptor agonist. Pharmacologically, cytisine exhibits a high degree of similarity to nicotine. Numerous studies have indicated that cytisine is useful in the treatment of nicotine addiction.

A pharmaceutical smoking cessation product containing cytisine has been commercialised for several years under the brand name Tabex^{®}. The Tabex^{®} product is marketed in the form of an orally administered tablet comprising 1.5mg of cytisine free base. While the product has been found to be efficacious and has been commercially successful, the approved shelf life of the product is two years.

As those skilled in the art will recognise, although a two year shelf life for a pharmaceutical product is generally acceptable, it does impose pressure on the manufacturer and supply chain in terms of promptly packaging, transporting and delivering the product to users, and also increases the risk of stock wastage if shelf-life is exceeded. Additionally, where the product is to be shipped to territories with higher temperature / humidity climates (e.g. ICH climactic zones III and IV), then additional packaging to protect the product and maintain shelf-life may be required.

A number of formulation approaches for cytisine-containing products are proposed in the prior art. For example, EP1586320 discloses a solid dosage formulation comprising cytisine free base. While it is stated in that document that the disclosed formulation provides improved stability, there is no suggestion that this could be achieved by the use of salts of cytisine. Indeed, no salts of cytisine are disclosed in that document.

WO2014/076680 discloses a cytisine-containing formulation which again is stated as improving the stability of cytisine. As with EP1586320, there is no suggestion of using a salt of cytisine to improve stability, nor are any salts mentioned. In WO2014/076680 the issue of incompatibility between the cytisine active ingredient and lactose is raised. Specifically, it is stated that lactose may destabilise tablets comprising cytisine due to the presence of a carboxyl group in the lactose molecule, which is not completely inert chemically and may lead to a Maillard reaction.

Accordingly, there is a need in the art for a form of cytisine which inherently is more compatible with conventional excipients such as lactose.

The present inventors have surprisingly and unexpectedly identified a novel salt of cytisine which displays improved excipient compatibility and can be formulated with lactose as an excipient.

Disclosed is a succinate salt of cytisine. In embodiments, the salt may be present in the form of a solvate or a hydrate. The salt is preferably cytisine hydrogen succinate.

As mentioned above, the improved compatibility of the salt permits the preparation of stable pharmaceutical compositions. Thus, disclosed is a pharmaceutical composition comprising the succinate salt of cytisine and a pharmaceutically acceptable carrier.

In an embodiment the pharmaceutically acceptable carrier is lactose. The lactose may be lactose monohydrate or anhydrous lactose.

The compositions disclosed herein may be suitable for administration by any route known in the art. Pharmaceutical compositions encompassed herein include those suitable for oral, nasal or topical administration. In an embodiment, the composition may be formulated in a solid form such as a tablet or a capsule.

In terms of excipients that may be employed in the compositions herein, these include fillers, disintegrants, preserving agents, lubricants (e.g. magnesium stearate) and / or wetting agents. Examples of fillers that may be used include lactose (either anhydrous or monohydrate), cellulose, starch (e.g. corn and / or wheat starch), calcium phosphates, mannitol and others known in the art.

Preserving agents prevent bacterial or fungal contamination of the formulation and may include various antibacterial and antifungal agents such as parabens, chlorobutanol, phenol or sorbic acid.

The pharmaceutical composition may be coated according to any method known in the art, for example using collidone or shellac, gum arabic, talc, titanium dioxide or sugar.

The pharmaceutical compositions may further comprise sweetening, flavouring or colouring agents.

In embodiments in which the pharmaceutical composition is provided in the form of capsules, these may be prepared by any suitable method. For example, such capsules may be prepared by mixing the salts with inert carriers such as lactose or sorbitol and packing them into gelatine capsules.

In embodiments, the pharmaceutical composition will be provided as a unit dosage form (e.g. a tablet, capsule). The amount of cytisine succinate salt in the composition may range from about 0.5mg or about 1.0mg to 2.0mg, 3.0mg, or about 5.0mg to about 10mg. In embodiments, the pharmaceutical compositions of the present invention may have a shelf life greater than 2 years when stored at 25°C and at a relative humidity of 60% ± 5%.

Embodiments of a unit dosage in accordance with the present disclosure are set out in the following clauses.
1. A unit dosage comprising a succinate salt of cytisine formulated as a pharmaceutical composition further comprising a pharmaceutically acceptable excipient.
2. The unit dosage of clause 1, wherein the succinate salt is cytisine hydrogen succinate.
3. The unit dosage of clause 1 or 2, wherein the succinate salt is a solvate or hydrate.
4. The unit dosage of any one of clauses 1 to 3, wherein the pharmaceutically acceptable excipient is selected from the group consisting of a filler, a disintegrant, a preserving agent, a lubricant, a wetting agent, and combinations thereof.
5. The unit dosage of any one of clauses 1 to 4, wherein the pharmaceutically acceptable excipient is a filler selected from the group consisting of lactose, corn starch, wheat starch, cellulose, calcium phosphate, and mannitol.
6. The unit dosage of any one of clauses 1 to 5, wherein the unit dosage comprises 0.5 mg to 10 mg of the succinate salt.
7. The unit dosage of any one of clauses 1 to 6, wherein the unit dosage comprises 1 mg to 10 mg of the succinate salt.
8. The unit dosage of any one of clauses 1 to 7, wherein the unit dosage comprises 2 mg to 10 mg of the succinate salt.
9. The unit dosage of any one of clauses 1 to 8, wherein the unit dosage comprises 3 mg to 10 mg of the succinate salt.
10. The unit dosage of any one of clauses 1 to 9, wherein the unit dosage comprises 5 mg to 10 mg of the succinate salt.
11. The unit dosage of any one of clauses 1 to 10, wherein the unit dosage comprises 5 mg of the succinate salt.
12. The unit dosage of any one of clauses 1 to 11, wherein the unit dosage is in a unit dosage form selected from the group consisting of an oral unit dosage form, a nasal unit dosage form, and a topical unit dosage form.
13. The unit dosage of any one of clauses 1 to 12, wherein the unit dosage is in a tablet or capsule form.
14. The unit dosage of any one of clauses 1 to 13, for use in treating nicotine addiction.

### Figures

Figure 1 shows HPLC chromatograms of cytisine at (A) 310nm and (B) 203nm.
Figure 2 shows HPLC chromatograms of succinic acid at (A) 310nm and (B) 203nm.
Figure 3 shows HPLC chromatograms of cytisine succinate at (A) 310nm and (B) 203nm.
Figure 4 shows the HR MS spectra of cytisine succinate: (A) ESI+ and (B) ESI-
Figure 5 shows the MS-MS spectra of cytisine succinate: (A) ESI+ and (B) ESI-
Figure 6 shows the IR spectra of cytisine succinate: (A) FTIR and (B) FTIR (ATR)
Figure 7 shows the peak purity determinations using a DAD detector for cytisine hydrogensuccinate (A) succinic acid and (B) cytisine.

The various embodiments of the present disclosure will now be further explained with reference to the following examples.

### Example 1: Preparation of cytisine succinate salt

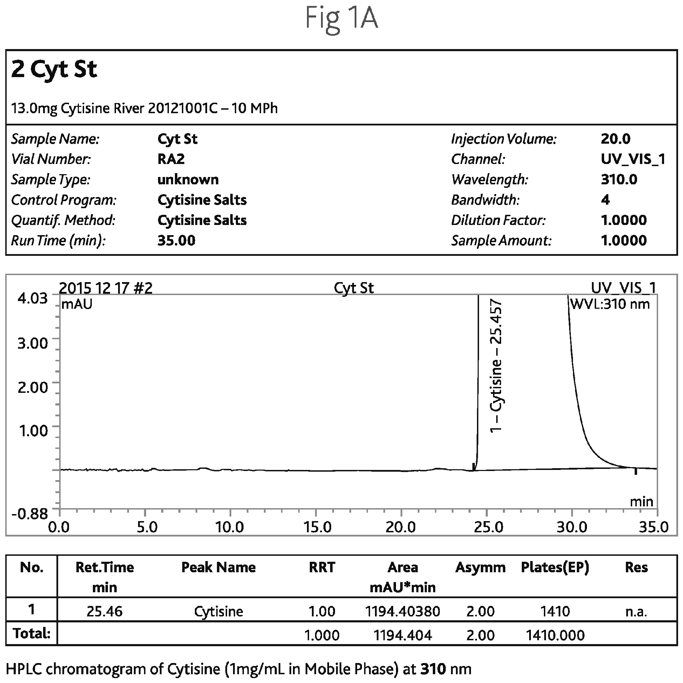

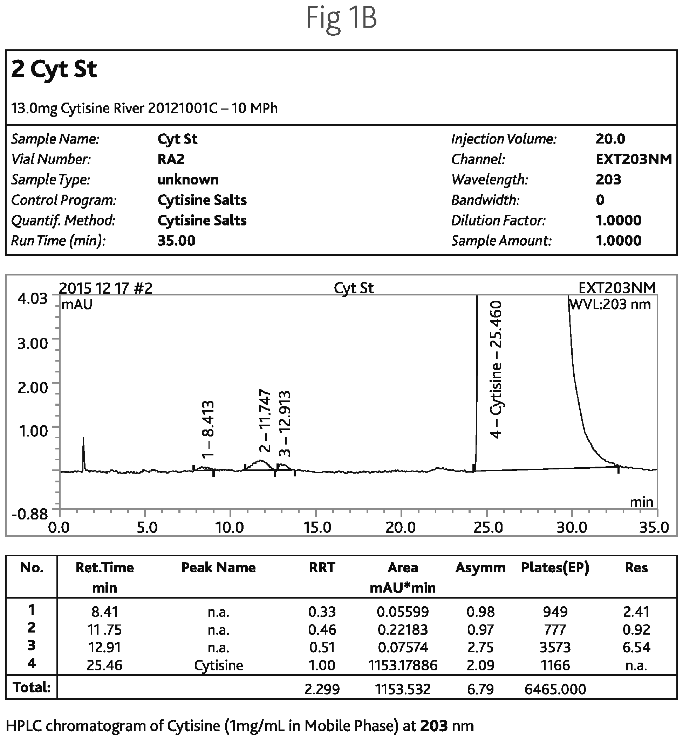

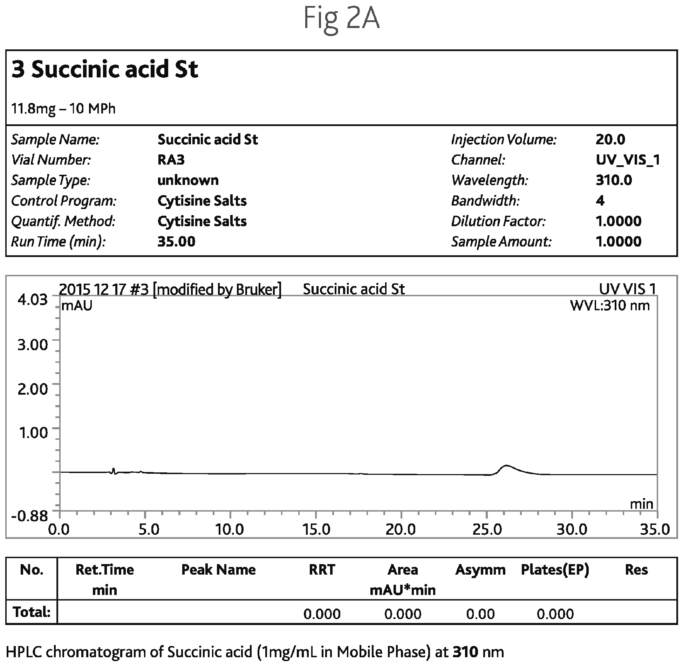

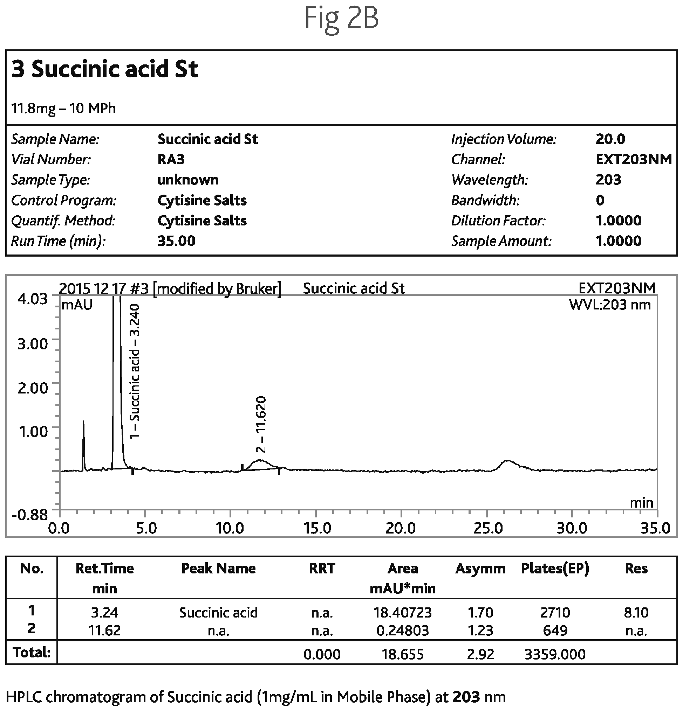

An aqueous mixture of cytisine and succinic acid was prepared. HPLC chromatograms for these starting materials are provided as Figures 1 and 2. Acetone was added, and cytisine succinate salt was isolated from the mixture at a yield of about 70%.

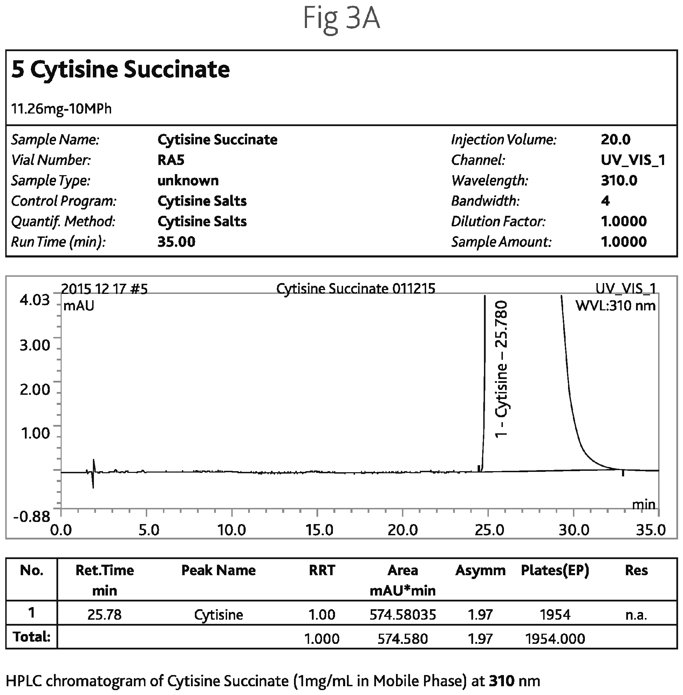

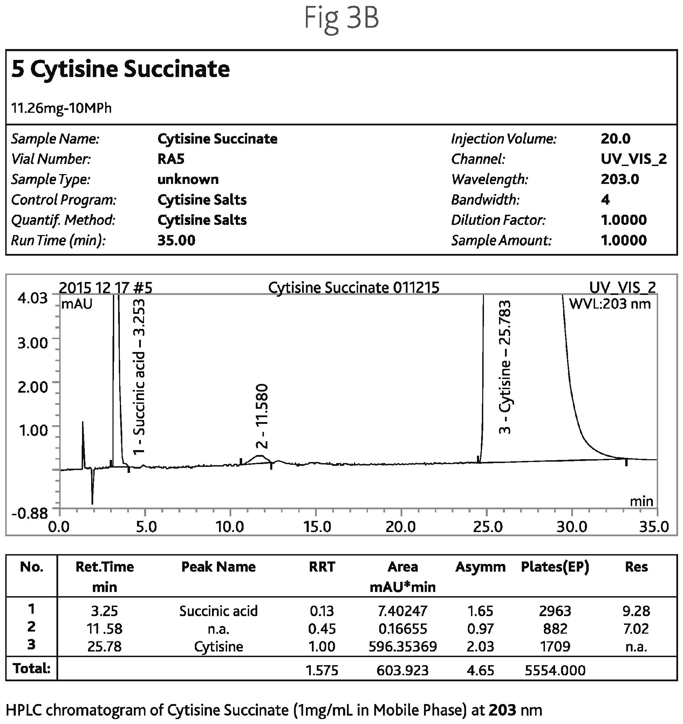

The starting materials (cytisine and succinic acid) and the obtained salt were characterised using a number of analytical methods, see Figures 1 to 7. The results are presented in Table 1 below. Chromatographic purity of the salt was determined using HPLC and was found to be greater than 99.9% (Figure 3). As can be seen, the salt of the present invention can be easily produced at very high levels of purity using conventional salification processes.

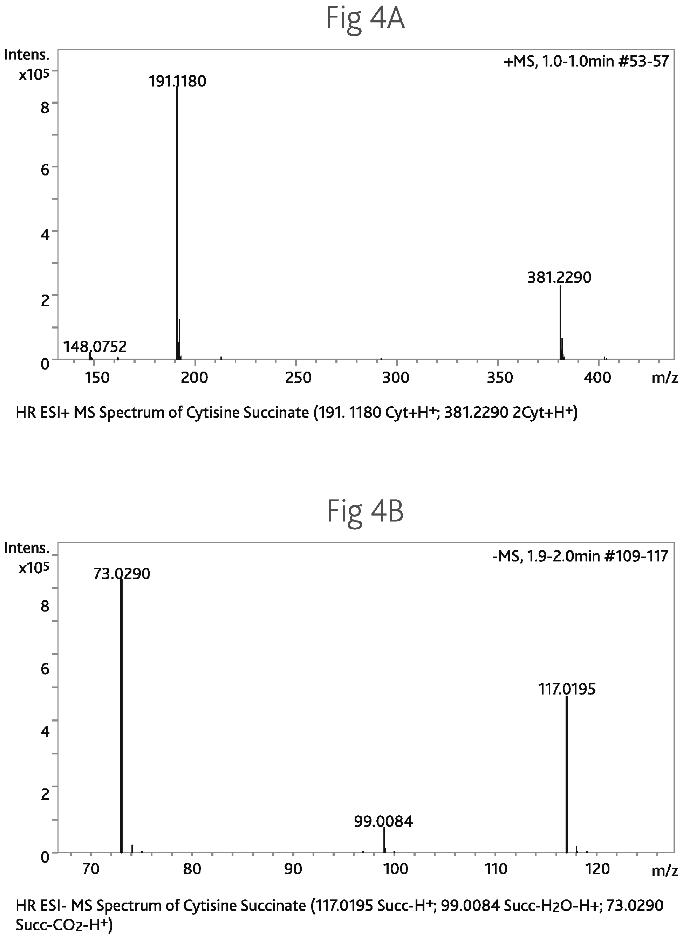

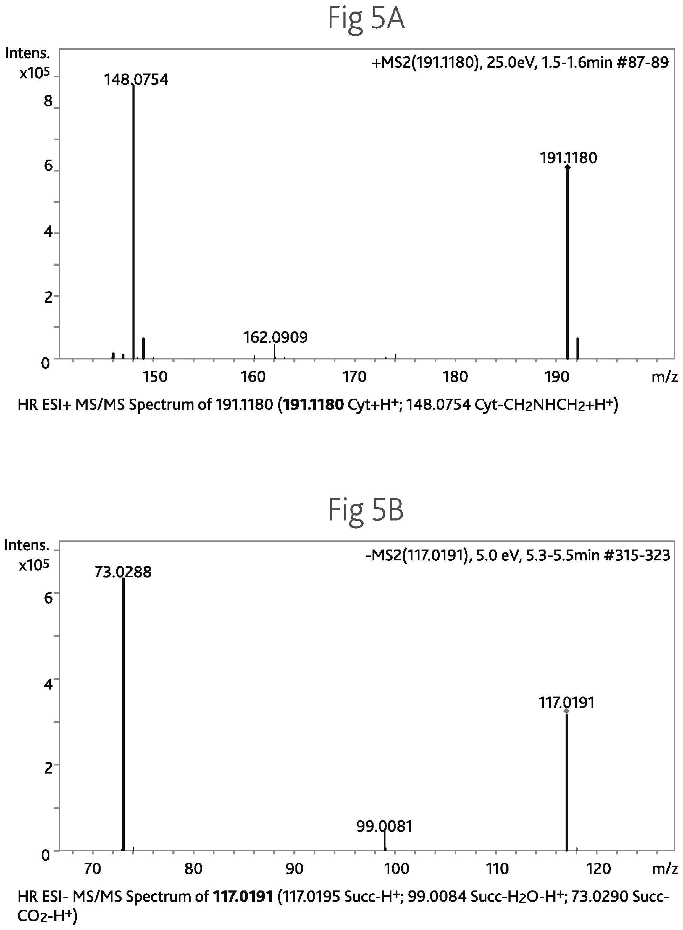

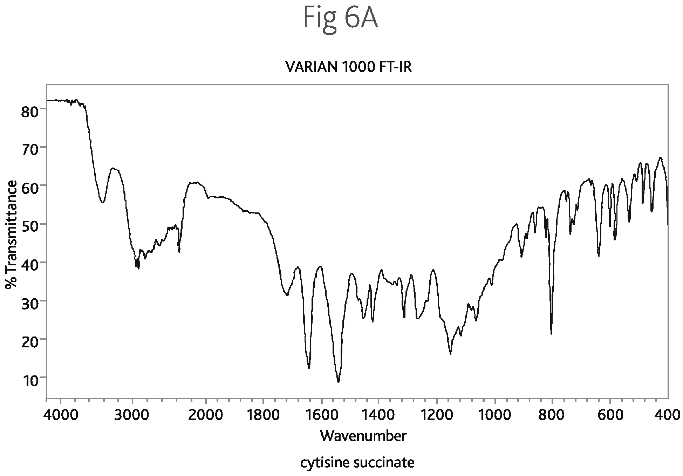

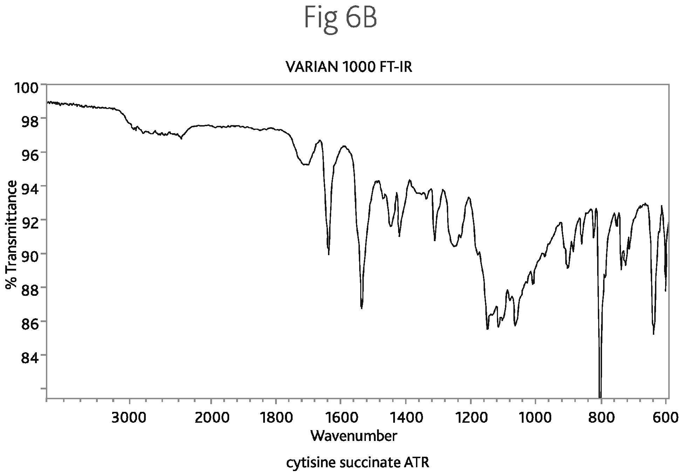

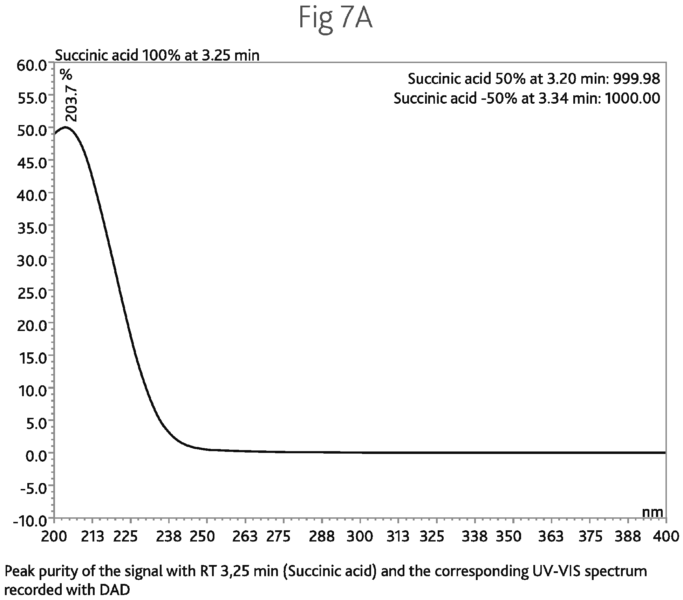

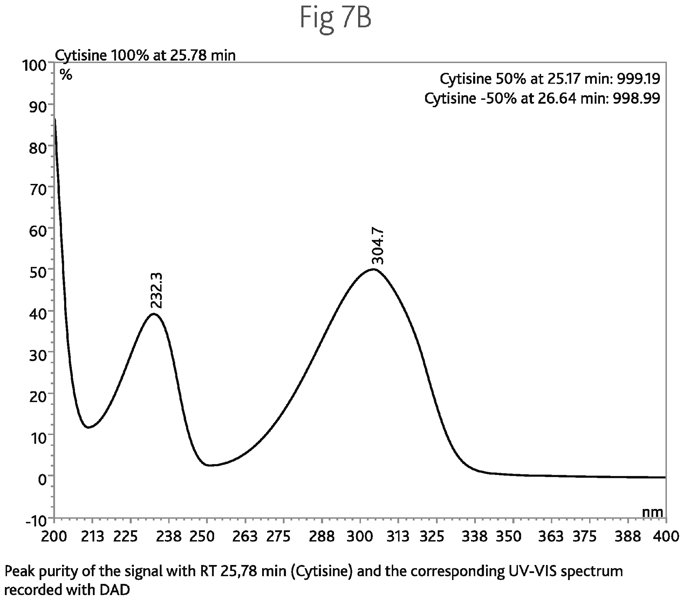

The obtained salt was also subjected to high resolution mass spectrum analysis (Figure 4), tandem mass spectroscopy (Figure 5), IR spectroscopy (Figure 6) and UV / VIS analysis (Figure 7) was performed.

**Table 1 Characterisation tests**

| No | Test items | Cytisine Succinate |
|---|---|---|
| 1 | Appearance | White crystals |
| 2 | UV/VIS (HPLC DAD) | |
| | UV/VIS Cytisine | Max. 232.3 nm; 304.7 nm |
| | UV/VIS Acid Anion | Max. 203.7 nm |
| 3 | IR | 2932 cm⁻¹; 2363 cm⁻¹; 1719 cm⁻¹; 1645 cm⁻¹; 1545 cm⁻¹; 1267 cm⁻¹; 1161 cm⁻¹; 805 cm⁻¹; 642 cm⁻¹ |
| 4 | HR MS (Q-TOF) | |
| | ESI+ | 191.1180 Cyt+H⁺ |
| | | 381.2290 2Cyt+H⁺ |
| | ESI- | 117.0195 Succ-H⁺ |
| | | 99.0084 Succ-H₂O-H⁺ |
| | | 73.0290 Succ-CO₂-H⁺ |
| 5 | Chromatographic purity (HPLC DAD) | 310 nm > 99.9% |
| | | 203 nm > 99.9% |

### Example 2: Stability of cytisine succinate salt

Samples of cytisine succinate salt obtained from Example 1 were formulated in standard formulations to investigate their stability, and the compatibility of the API with the excipients used. Preliminary analysis of these formulations indicates that the formulations comprising succinate salt are significantly more stable than those comprising cytisine free base.

### Example 3 - Salt Screen

Stock solutions of acid salt formers were prepared in the carrier solvents and having the molarities detailed in the following table:

| Acid | Solvent mix | Molarity |
|---|---|---|
| Acetate | EtOH | 1 |
| Ascorbate | 9:1 EtOH:water | 0.5 |
| Benzoate | EtOH | 1 |
| Succinate | EtOH | 0.5 |

Stock solutions of cytisine API (non-synthetic) were also prepared in CH₃CN and 2-Me-THF. 2.4g of cytisine was dissolved in 24ml of CH₃CN heated to a temperature of 40°C. 2.4g of cytisine was dissolved in 60ml of 2-Me-THF heated to a temperature of 60°C.

10ml capacity tubes heated to 40°C were charged with 2ml of the cytisine / CH₃CN stock solution or 5ml of the cytisine 2-Me-THF stock solution. The acid stock solutions were then added to the heated tubes in equimolar amounts. The solutions were held at 40°C for one hour and then allowed to cool to ambient temperature (~18°C) for 18 hours. Where solid formation did not spontaneously occur, manipulations were carried out, namely: i) gradual blow down under nitrogen to induce crystallisation, ii) charging antisolvent / triturate, and iii) second blow down under nitrogen and trituration with 3ml TBME and 1ml acetone.

Filtration of the obtained solids was then carried out using a PTFE fritted column and the obtained solids were dried at 50°C for 48 hours. The properties of the obtained products were then analysed, and the following table summarises the outcome of this screen and the properties of the obtained products:

| API Stock Solution | Salt-type | Assessment |
|---|---|---|
| CH₃CN | Acetate | Base precipitation, unstable salt |
| 2-MeTHF | Acetate | Base precipitation, unstable salt |
| CH₃CN | Ascorbate | Base precipitation, unstable salt |
| 2-MeTHF | Ascorbate | Base precipitation, unstable salt |
| CH₃CN | Benzoate | Failed to yield solid |
| 2-MeTHF | Benzoate | Failed to yield solid |
| CH₃CN | Succinate | Excellent salt profile, mono succinate, reasonably high melt |
| 2-MeTHF | Succinate | Equivalent succinate to CH₃CN example with phase impurity/thermally induced modulation, possible polymorphism |

As can be seen, suitable salts could not be formed with the common acid salt formers acetic acid, ascorbic acid or benzoic acid. However, the succinate salt was readily formed, exhibiting advantageous properties.

### Example 4 - Lactose Incompatibility Testing

Cytisine, 0.9541g, was dissolved in water, 1ml, and afforded a yellow solution. Succinic acid, 0.5919g, 1equiv, was charged as a solid to the cytisine solution and dissolved slowly with agitation. Acetone, 10ml, was charged and afforded a partitioned mixture of cytisine/succinic acid/water solution, lower, and acetone, upper. Trituration of a portion of cytisine/succinic acid/water solution with acetone, 10ml, converted the viscous mixture to a white solid which settled. The white suspension was charged to the remainder of the cytisine/succinic acid/water solution/acetone mixture with a rinse of acetone, 10ml, and agitation continued. This converted the viscous cytisine/succinic acid/water mixture to a white suspension which settled when agitation was stopped. The solid was isolated by filtration and dried in vacuo at 50°C for ca 16 hours. The recovered solid was confirmed as being cytisine succinate by ¹H NMR analysis.

Recovery: 1.5463g, 80.76% based upon a salt stoichiometry of cytisine to succinic acid of 1:1
The stability of cytisine / lactose and cytisine succinate / lactose binary mixtures was assessed at 40°C and 75% relative humidity (RH) in vials with loosened lids at 9 days.

The sample mixtures and storage conditions used are detailed in Table 2 and Table 3.

**Table 2 Cytisine /lactose mixtures and storage conditions**

| No. | Cytisine, mg | Lactose, mg | Storage condition |
|---|---|---|---|
| 1 | 100.6 | 99.8 | 40°C and 75% RH |

**Table 3 Cytisine succinate/lactose mixtures and storage conditions**

| No. | Cytisine succinate, mg | Lactose, mg | Storage condition |
|---|---|---|---|
| 2 | 98.4 | 101.8 | 40°C and 75% RH |

The chemical purity of a cytisine/lactose mixture was 99.83 area% and a cytisine succinate/lactose mixture was 99.68 area% at the start of the of the stability study.

Upon completion of the stability testing period, the following results were observed:

**Table 4 Characteristics of cytisine, cytisine succinate, cytisine/lactose and cytisine succinate/lactose stored for 9 days at 40°C and 75% RH**

| No. | CP of cytisine by HPLC, area % |
|---|---|
| 1 - Cytisine/ lactose | 62.75 |
| 2 - Cytisine succinate/lactose | 77.47 |

As can be seen from the data in Table 4, when present in the form of its succinate salt, cytisine is degraded at a substantially lower rate than when present in free base form. Thus, the succinate salt effectively improves the stability of cytisine and facilitates its formulation with compositions comprising lactose.

## Claims

1. A hydrogen succinate salt of cytisine, wherein the hydrogen succinate salt of cytisine has enhanced degradation resistance relative to cytisine in free base form.

2. A pharmaceutical composition comprising the hydrogen succinate salt of claim 1 and a pharmaceutically acceptable excipient.

3. The pharmaceutical composition according to claim 2, wherein the composition is in the form of a tablet or capsule.

4. The pharmaceutical composition according to claim 2 or 3, wherein the pharmaceutically acceptable excipient is selected from the group consisting of lactose, cornstarch, wheat starch, and mixtures thereof.

5. The pharmaceutical composition according to any one of claims 2-4, wherein the composition is provided as a unit dosage form.

6. The pharmaceutical composition according to claim 5, wherein the unit dosage form comprises 1 to 3 mg of cytisine hydrogen succinate.

7. The pharmaceutical composition of any one of claims 2-6, for use in the treatment of nicotine addiction.

8. Use of a hydrogen succinate salt of cytisine in the manufacture of a medicament for the treatment of nicotine addiction, wherein the hydrogen succinate salt of cytisine has enhanced degradation resistance relative to cytisine in free base form.

9. The use of claim 8, wherein the medicament comprises 1 to 3 mg of the hydrogen succinate salt of cytisine.

10. The use of any one of claims 8 or 9, wherein the medicament is in the form of a tablet or capsule.
